# EUROPEAN PATENT SPECIFICATION

(11) **EP 2 159 577 B1**
(45) Date of publication and mention of the grant of the patent: **08.08.2012**
(21) Application number: 08764122.1
(22) Date of filing: 13.06.2008
(51) Int. Cl.: G01N 33/92, C07K 16/28, C12N 5/16

(54) **NOVEL MARKER FOR ARTERIOSCLEROTIC DISEASE**
NEUER MARKER FÜR ARTERIOSKLEROSEKRANKHEIT
NOUVEAU MARQUEUR D'UNE MALADIE ARTÉRIOSCLÉROTIQUE

(30) Priority: 18.06.2007 JP 2007160225
(43) Date of publication of application: 03.03.2010
(73) Proprietor: Sekisui Medical Co., Ltd., Tokyo 103-0027 (JP)
(72) Inventor: MATSUO, Masanao, Ryugasaki-shi Ibaraki 301-0852 (JP); EBINUMA, Hiroyuki, Ryugasaki-shi Ibaraki 301-0852 (JP); FUKAMACHI, Isamu, Ryugasaki-shi Ibaraki 301-0852 (JP); BUJO, Hideaki, Chiba-shi Chiba 260-0852 (JP); SAITO, Yasushi, Chiba-shi Chiba 260-0853 (JP)
(74) Representative: Hartz, Nikolai
(86) International application number: PCT/JP2008/001527
(87) International publication number: WO 2008/155891

(56) References cited:
- WO-A2-01/64874
- WO-A2-2005/117561
- OHWAKI KENJI ET AL: "A secreted soluble form of LR11, specifically expressed in intimal smooth muscle cells, accelerates formation of lipid-laden macrophages" 1 May 2007 (2007-05-01), ARTERIOSCLEROSIS, THROMBOSIS, AND VASCULAR BIOLOGY, LIPPINCOTT WILLIAMS & WILKINS, US LNKD- DOI:10.1161/ATVBAHA.106.137091, PAGE(S) 1050 - 1056 , XP009134179 ISSN: 1079-5642 [retrieved on 2007-03-01] * the whole document *
- OHWAKI KENJI ET AL: "Identification of a novel regulator of lipid accumulation of macrophages secreted from intimal smooth muscle cells" 31 October 2006 (2006-10-31), CIRCULATION, LIPPINCOTT WILLIAMS & WILKINS, US, PAGE(S) 252 - 253 , XP009134180 ISSN: 0009-7322 * abstract *
- ZHU YANJUAN ET AL: "LR11, an LDL receptor gene family member, is a novel regulator of smooth muscle cell migration" 1 April 2004 (2004-04-01), CIRCULATION RESEARCH, AMERICAN HEART ASSOCIATION, INC, US LNKD- DOI:10.1161/01.RES.0000120862.79154.0F, PAGE(S) 752 - 758 , XP009134177 ISSN: 1524-4571 [retrieved on 2004-02-05] * abstract *
- TAKAHASHI MAO ET AL: "Circulating levels of soluble form of LR11, a novel migration regulator of intimal SMC, are increased in subjects with coronary stenosis" 11 March 2008 (2008-03-11), JOURNAL OF THE AMERICAN COLLEGE OF CARDIOLOGY, ELSEVIER, NEW YORK, NY, US, PAGE(S) A296 , XP009134183 ISSN: 0735-1097 * the whole document *
- PANG MEIZI ET AL: "A soluble form of Ldl receptor gene family member, Lr11, is essential for angiotensin II-induced Smc migration" 1 October 2007 (2007-10-01), CIRCULATION, LIPPINCOTT WILLIAMS & WILKINS, US, PAGE(S) 298 , XP009134182 ISSN: 0009-7322 * the whole document *
- MATSUO MASANAO ET AL: "Development of an immunoassay for the quantification of soluble LR11, a circulating marker of atherosclerosis" 1 October 2009 (2009-10-01), CLINICAL CHEMISTRY, AMERICAN ASSOCIATION FOR CLINICAL CHEMISTRY, WASHINGTON, DC, PAGE(S) 1801 , XP009134189 ISSN: 0009-9147
- KENJI OHWAKI ETAL.: 'A secreted soluble from of LR11,l specifically expressed in intimal smooth muscle cells, accelerated formation of lipid-laden macrophages' ARTER. THROMB. VASC. BIOL. vol. 27, no. 5, 16 May 2007, pages 1050 - 1056, XP009134179
- YANJUAN ZHU ET AL.: 'LR11, an LDL receptor gene family member, is a novel regulator of smooth muscle cell migration' CIRCULAT. RES. vol. 94, no. 6, 2004, pages 752 - 758, XP009134177
- TAKESHIRO H.: 'Kekkan no Lipidology LDL Juyotai Family to Kekkan' VASCULAR BIOLOGY & MEDICINE vol. 5, no. 5, 2004, pages 469 - 474

### SEQUENCE LISTING

<110> Sekisui Medical Co., Ltd.
<120> A Novel Marker of Arteriosclerotic Disease
<130> DC0065
<150> JP2007-160225
   <151> 2007-06-18
<160> 1
<170> PatentIn version 3.1
<210> 1
   <211> 16
   <212> PRT
   <213> Artificial Sequence
<220>
   <223> Synthetic peptide based on LR11
<400> 1
   Ser Met Asn Glu Glu Asn Met Arg Ser Val Ile Thr Phe Asp Lys Gly
   1               5                   10                  15

## Description

### Technical Field

The present invention relates to a method for evaluating the presence or level of an arteriosclerotic disease and to a method for evaluating a prophylactic or therapeutic effect on the disease.

### Background Art

Arteriosclerotic diseases, including arteriosclerosis, are often accompanied by cerebral infarction, cerebral hemorrhage, aortic aneurysm, myocardial infarction, etc. with the progress of an arteriosclerotic lesion. Therefore, evaluation of the presence or level of an arteriosclerotic disease and prevention and treatment thereof are very important. In conventional clinical examinations, markers such as total blood cholesterol, LDL-cholesterol, HDL-cholesterol, neutral fat, and remnant-like lipoprotein-cholesterol have been widely measured.

However, even by the measurements of these markers, the presence or level of arteriosclerosis or a prophylactic or therapeutic effect on arteriosclerosis has not been satisfactorily evaluated. Thus, there is demand for a novel marker for evaluating the presence or level of an arteriosclerotic disease.

LR11 (LDL receptor relative with 11 ligand-binding repeats) was previously identified as a novel LDL receptor-like protein having a characteristic structure of the LDL receptor family (Patent Document 1 and Non-Patent Document 1). At that time, SorLA (sorting protein-related receptor containing LDL receptor class a repeats) was isolated from a brain tissue through RAP (The 39-40 kDa receptor-associated protein) affinity chromatography in another study (Non-Patent Document 2), which is the same protein as LR11. LR11 is known to have 11 ligand-binding repeats which recognize apo E as a common ligand, and is not expressed in normal vascular wall cells but expressed specifically in smooth muscle cells in thickened intima (Non-Patent Document 3). A study by use of cultured smooth muscle cells revealed that expression of LR11 was promoted in response to proliferation of smooth muscle cells, leading to secretion of LR11 to the culture solution, and a study by use of a cuff injury mouse model revealed that intraperitoneal administration of an anti-LR11 antibody inhibited thickening of vascular intima which would otherwise be caused by migration and proliferation of smooth muscle cells (Non-Patent Document 4). These studies suggest that LR11 can be employed as an index for proliferation of vascular smooth muscle cells. Meanwhile, there have been known substances which can bind to LR11, such as apolipoprotein E (apo E), apo E-rich VLDL (β-VLDL), RAP aforementioned, uPA (urokinase-type plasminogen activator), PAI-1 (type-1 plasminogen activator inhibitor), and a complex thereof (uPA-PAI-1) (Non-Patent Document 5).

However, LR11 is a protein which intrinsically expresses on cell surfaces, and soluble LR11 has never been reported to be present in a sample collected from a mammal, particularly in a blood sample. No attempt has been made to apply soluble LR11 to evaluation of the presence or level of an arteriosclerotic disease or to assessment of the effect thereof on prophylaxis and treatment of the disease.
Patent Document 1: JP-A-1997-163988
Non-Patent Document 1: J. Biol. Chem. 1996; 271, 24761-24768
Non-Patent Document 2: J. Biol. Chem. 1996; 271, 31379-31383
Non-Patent Document 3: Arterioscler. Thromb. Vasc. Biol. 1999; 19, 2687-2695
Non-Patent Document 4: Circ. Res. 2004; 94; 752-758
Non-Patent Document 5: Biochem. J. 2004; 381, 203-212
Ohwaki et al., Arteriosclerosis, Thrombosis, and Vascular Biology 2007, 27, 1050-1056 describes that a secreted soluble form of LR11 is expressed in intimal smooth muscle cells and accelerates formation of lipid-laden macrophages. WO 01/64874 relates to a low density lipoprotein binding protein (LBP) and suggests a method for determining a risk for atherosclerosis by evaluating an aspect of LBP metabolism or structure. Ohwaki et al., Circulation 2006, 114, II_252_II_253 describes identification of a novel regulator of lipid accumulation of macrophages secreted from intimal smooth muscle cells.

### Disclosure of the Invention

### Problems to be Solved by the Invention

An object of the present invention is to provide a novel marker for arteriosclerotic diseases.

### Means for Solving the Problems

The present inventors have carried out studies on the presence of LR11 in blood by use of a substance having an affinity to LR11 and through extraction of soluble LR11 in blood by use of a RAP-bearing resin, and have found that although no substantial expression of LR11 is observed in normal vascular wall cells, quite surprisingly, soluble LR11 is present in a blood sample derived from a normal mammal, particularly in a blood sample collected from a healthy human subject. Another study by the inventors has also revealed that the molecular weight of the extracted soluble LR11 is slightly smaller than that of LR11 protein expressed on cell surfaces. Therefore, soluble LR11 is thought to have a structure lacking a C-terminal membrane-spanning domain. Based on these findings, the present inventors have conducted further studies, and found that the level of soluble LR11 in blood samples from arteriosclerotic disease patients is significantly higher than that from healthy subjects and thereby accomplished the present invention.

Accordingly, the present invention provides the following:
(1) a method for evaluating the presence or level of an arteriosclerotic disease in a mammal or for evaluating a prophylactic or therapeutic effect on an arteriosclerotic disease in a mammal, including detecting soluble LR11 in a sample collected from the mammal, wherein the sample is blood, serum or plasma;
(2) the method according to (1), wherein the mammal is a human;
(3) the method according to (1) or (2), wherein the soluble LR11 is a protein having a structure of LR11 in which a C-terminal membrane-spanning domain is deficient;
(4) the method according to any of (1) to (3), wherein the soluble LR11 is detected by use of a substance having an affinity thereto;
(5) the method according to (4), wherein the substance having an affinity to the soluble LR11 is an anti-soluble LR11 antibody and/or RAP;
(6) a use of a kit for evaluating the presence or level of an arteriosclerotic disease or for evaluating a prophylactic or therapeutic effect on an arteriosclerotic disease, said kit comprising a substance which detects soluble LR11 and has affinity to soluble LR11.
Described is a kit for evaluating the presence or level of an arteriosclerotic disease or for evaluating a prophylactic or therapeutic effect on an arteriosclerotic disease, including a substance which detects soluble LR11;
wherein the substance which detects soluble LR11 may be an anti-soluble LR11 antibody and/or RAP.

### Effects of the Invention

By the present invention, it was elucidated that LR11, which promotes thickening of vascular smooth muscle cells, is present in the form of soluble LR11 in blood of a human and a mammal. The present invention realizes evaluation of the presence or level of arteriosclerotic disease in the early stage through measuring the blood soluble LR11 level not only in an arteriosclerotic disease patient but also in a healthy subject. The invention also realizes monitoring of a prophylactic or therapeutic effect of a drug or the like on the progress of arteriosclerosis in an arteriosclerotic disease patient.

### Brief Description of the Drawings

[Fig. 1] A chart showing the results of western blotting analysis of soluble LR11 contained in serum samples of normal mammals performed in Example 3.
[Fig. 2] A chart showing the results of western blotting analysis of soluble LR11 in a human serum and LR11 derived from human neuroblasts (IMR32), performed in Example 4.

### Best Modes for Carrying Out the Invention

In the present invention, the term "arteriosclerotic disease" is intended to include a disease caused by the progress of a lesion of an arteriosclerotic disease. No particular limitation is imposed on such a disease, and examples of the disease include myocardial infarction, restenosis occurring after percutaneous coronary intervention, cerebral infarction, cerebral hemorrhage, aortic aneurysm and obliterative arteriosclerosis. The term "evaluation of the presence or level" refers not only to evaluation of a patient who has already suffered an arteriosclerotic disease, but also to determining whether or not a subject is likely to suffer an arteriosclerotic disease in the future.

No particular limitation is imposed on a subject mammal in the present invention, so long as the mammal produces soluble LR11. Examples of the mammal include human, monkey, goat, sheep, pig, bovine, mouse, rabbit and rat. Of these, human is preferred. The bio-sample derived from a mammal is blood, serum or plasma. Of these, serum and plasma are preferred.

The soluble LR11 contained in a bio-sample is detected by use of a substance having an affinity to soluble LR11. No particular limitation is imposed on the substance having an affinity to soluble LR11, so long as the substance is capable of binding to soluble LR11. Examples of the substance include apo E, β-VLDL, RAP, uPA, PAI-1, a complex thereof (uPA-PAI-1) and an anti-soluble LR11 antibody. Of these, RAP and an anti-soluble LR11 antibody are preferred, with an anti-soluble LR11 antibody being more preferred.

The anti-soluble LR11 antibody may be a polyclonal antibody or a monoclonal antibody, and can be produced through a method well-known in the art. Commercially available products of these antibodies may also be employed in the present invention. Examples of such commercial antibodies include Mouse anti-LR11 monoclonal antibody (product of BD biosciences) and Rabbit anti-LR11 Polyclonal antibody (product of CHEMICON).

The immunogen for producing an anti-LR11 antibody employed in the invention may be LR11 protein, a partial fragment thereof (peptide), etc. The employable immunogen may be obtained through purification of a bio-sample, or a recombinant protein.
The animal immunized by the immunogen includes mouse, rat, hamster, rabbit, goat, sheep and chicken. The immunization is performed in accordance with a known method. In one exemplified procedure, a suspension of an immunogen in a commonly used buffer or physiological saline, or a mixture of an immunogen with an adjuvant such as complete Freund's adjuvant is administered subcutaneously, intradermally or intraperitoneally to an animal for primary stimulation. The stimulation is optionally repeated. The amount of immunogen administered to the animal is appropriately determined depending on the administration route and the kind of the animal.

In the case of polyclonal antibody, an anti-serum containing the polyclonal antibody may be prepared through housing an immunized animal for a predetermined period of time and collecting blood from the animal. The monoclonal antibody may be produced through a known method for producing a monoclonal antibody; e.g., "Monoclonal Antibody" (Hideaki NAGAMUNE and Hiroshi TERADA, Hirokawa-shoten, 1990) or "Monoclonal Antibody" (Jame W. Golding, 3rd edition, Academic Press, 1996).

The monoclonal antibody may be produced through, for example, a method including culturing hybridomas produced by a commonly-used method and isolating the antibody from the culture supernatant; or a method including administering a hybridoma to a mammal compatible with the hybridoma and collecting the antibody with ascites fluid from the mammal.
If required, the antibody may be further purified before use. Examples of the antibody purification/isolation method include known methods such as salting out (e.g., ammonium sulfate precipitation), gel filtration (e.g., by use of Sephadex), ion-exchange chromatography and affinity purification (e.g., by use of protein A column).

No particular limitation is imposed on the method for detecting soluble LR11 contained in a sample collected from a mammal. However, preferably, an immunological method employing an anti-soluble LR11 antibody, a method employing affinity of RAP or the like, or a combination method thereof is employed for detection. Examples of the immunological method include immunostaining (western blotting), enzyme-linked immunosorbent assay (ELISA), immunonephelometry (TIA or LTIA), enzyme immunoassay, chemiluminescent immunoassay, and fluorescent immunoassay. Alternatively, sandwich ELISA employing an antibody and a substance having an affinity to LR11 (e.g., RAP) may also be employed. In a yet alternative method, soluble LR11 contained in a sample is caused to be adsorbed by an insoluble carrier bearing a substance having an affinity to LR11 (e.g., RAP), and the adsorbed matter is washed and eluted by use of an appropriate buffer. Such a preliminary treatment is preferred to remove contaminating proteins in the sample and thereby resulting in precise soluble LR11 assay.
In order to quantitatively or semi-quantitatively evaluate the detected soluble LR11, comparison with reference LR11 is preferably carried out. In this case, the reference LR11 is preferably, for example, serum soluble LR11 having a known concentration, LR11 recovered from cultured cells or culture supernatant of smooth muscle cells or neuroblasts, recombinant LR11, or a synthetic peptide used as an immunogen in preparation of the antibody.

### (Example of determination of LR11 in a human body fluid sample)

The following is an example of determination of LR11 in a human body fluid sample according to the present invention. Firstly, a human body fluid sample is allowed to react with an insoluble carrier bearing RAP or an anti-LR11 monoclonal antibody which has an affinity to LR11. Subsequently, the insoluble carrier is treated with SDS under reducing conditions, followed by centrifugation. The centrifugation supernatant is subjected to SDS-polyacrylamide gel electrophoresis (SDS-PAGE). Through a commonly-used western blotting technique, the protein is transferred to a PVDF membrane, and the membrane is reacted with the anti-LR11 monoclonal antibody and then with a labeled (e.g., enzyme-labeled) anti-mouse IgG antibody. Thereafter, the reaction is detected through western blotting.

According to one preferable embodiment of the method of the present invention for evaluating the presence or level of an arteriosclerotic disease, a bio-sample is collected from a mammal suspected of having an arteriosclerotic disease, and the level of the soluble LR11 in the sample is determined. Separately, a bio-sample is collected in a similar manner from the group of healthy mammals not suspected of having an arteriosclerotic disease, and the level of the soluble LR11 in the sample is calculated. The two LR11 levels are compared with each other, whereby the presence or level of an arteriosclerotic disease is evaluated. In an alternative embodiment, bio-samples are collected at multiple time points from a mammal suffering from an arteriosclerotic disease, and the time-dependent change in soluble LR11 level is monitored, whereby the therapeutic effect can be evaluated. The evaluation can be performed on the basis of the change in soluble LR11 level. For example, an increase trend is thought to indicate progress of the arteriosclerotic disease, and a decrease or stable trend is thought to indicate suppression of progress of the arteriosclerotic disease.
As used herein, the term "healthy" refers to an individual having no arteriosclerotic disease. The presence or absence of an arteriosclerotic disease of human subjects is objectively determined by the following criteria:
no history of an arteriosclerotic disease such as coronary occlusion, and, for example,
the blood pressure falling within normal range (systolic pressure: <130 mmHg, diastolic pressure: <85 mmHg) according to "Guideline for the Management of Hypertension 2004," by The Japanese Society of Hypertension;
the blood sugar level falling within normal range (fasting blood sugar: <110 mg/dL) according to the committee report regarding classification and diagnostic standards of diabetes (1999) defined by Japanese Diabetes Society; and
the serum lipid level not falling within abnormal range (i.e., total cholesterol: <220 mg/dL, LDL cholesterol <140 mg/mL, HDL cholesterol ≥40 mg/dL and triglyceride: <150 mg/dL, in blood collected at fasting) based on the diagnostic standards of hyperlipidemea (2002), by Japan Atherosclerosis Society.
Another advantageous embodiment of the present invention includes detection of soluble LR11 and one or more other arteriosclerotic disease-related markers and combining the results obtained by the markers, in order to more accurately determine the progress of an arteriosclerotic lesion, the morbidity risk for an arteriosclerotic disease, and the therapeutic effect on a drug or the like. Examples of such markers other than LR11 include known markers, such as LDL-cholesterol, HDL-cholesterol, oxidized LDL, small particle LDL, remnant-like lipoprotein-cholesterol, neutral fat, an inflammation marker such as CRP, and adipocytokines (e.g., adiponectin, PAI-1, and TNF-α). These markers can be detected through a corresponding known detection method. Preferably, the determination is carried out, for example, with reference to the reference range specific to each marker. The level of markers including a soluble LR11 level may be examined individually or in combination with the level of two or more items. Examples of the combined examination include evaluating the level of soluble LR11 in association with the level of another marker. Specific examples of a method for such associative evaluation include a method employing, as an index, the conditions where the soluble LR11 level and the level of another marker are both in excess of the reference range; a method of determining a relative value (soluble LR11/another marker or another marker/soluble LR11); and a method of calculating the total score of the soluble LR11 score and the score of another marker after scoring each of those markers. The type and number of markers other than LR11 to be combined with LR11 may be appropriately selected depending on factors to be focused such as the purpose of evaluation of the presence or level of the marker and accuracy in detection.

The kit for evaluating the presence or level of an arteriosclerotic disease or for evaluating a prophylactic or therapeutic effect on an arteriosclerotic disease, contains a substance which is capable of detecting soluble LR11. No particular limitation is imposed on the substance which is capable of detecting soluble LR11, provided it has affinity to soluble LR11, such as an anti-soluble LR11 antibody or RAP. The kit of the invention may further include an additional component required for detecting soluble LR11, such as a reaction buffer and a reaction vessel.

### Examples

The present invention will be described in detail hereinafter by way of examples, which should not be construed as limiting the invention thereto.

### Example 1: Production of anti-LR11 antibody

### (1) Preparation of hybridomas

A cysteine residue was introduced to the C-terminal of a synthetic peptide of a partial amino acid sequence of LR11 [432-447] (SMNEENMRSVITFDKG), and KLH (keyhole lympet hemocyanin) was coupled, via a lysine residue therein, to the peptide by use of a cross-linking agent (NBS, product of PIERCE), to thereby produce an immunogen. The immunogen was admixed with complete Freund's adjuvant (product of GIBCO) at 1 : 1 to form an emulsion. The immunogen (0.1 mg/0.1 mL emulsion) was subcutaneously administered to 6-week-old female BALB/C mice 10 times at intervals of one week. Three days after the final immunization, the spleen was removed from each mouse. The splenic cells obtained from the removed spleen were mixed with myeloma cells SP2/O-Ag14 at 6 : 1, and the two kinds of cells were fused in the presence of 50% polyethylene glycol 1540 (product of Wako Pure Chemical Industries, Ltd.). The fused cells were suspended in a HAT medium such that the splenic cell concentration was adjusted to 2.5 × 10⁶/mL, and the suspension was dispensed to a 96-well culture plate (product of CORNING) at 0.2 mL/well. The cells were cultivated in a 5%CO₂ incubator at 37°C. About two weeks after the start of the cultivation, wells in which hybridomas were grown were selected, and the culture supernatant of each of the selected wells was analyzed through the ELISA method as described below. The presence of an antibody to the synthetic peptide of a partial amino acid sequence of LR11 [432-447] (SMNEENMRSVITFDKG) was determined to select a hybridoma promising to produce a target antibody.

Specifically, the synthetic peptide of a partial amino acid sequence of LR11 [432-447] (SMNEENMRSVITFDKG) was immobilized on a micro-plate (product of NUNC). The peptide was reacted with IgG from each culture supernatant, and further reacted with a peroxidase-labeled anti-mouse IgG goat antibody. Subsequently, a peroxidase substrate solution containing o-phenylenediamine (product of Tokyo Chemical Industry Co., Ltd.) was added to the reaction solution, to thereby allow the solution to develop color. The color development was stopped by addition of 1.5N sulfuric acid, and the absorbance at 492 nm was measured by means of a micro-plate reader. As a result, a hybridoma which exhibited reactivity to the aforementioned synthetic peptide was selected. The hybridoma was cloned through limiting dilution analysis. The ultimate reactivity to LR11 was confirmed by recovering LR11 contained in a culture supernatant of the below-described human neuroblast cells (IMR32) through adsorbing LR11 onto RAP-Sepharose resin, performing electrophoresis, transferring the LR11 to a PVDF membrane, and performing western blotting analysis. Thus, an antihuman LR11 monoclonal antibody-producing hybridoma (A2-2-3) was established.

### (2) Preparation of monoclonal antibody

Pristane (0.5 mL) was intraperitoneally injected 12-week-old female BALB/C mice. Two weeks after, the above-established hybridoma (0.5 x 10⁶ cells) was intraperitoneally injected to each of the mice. About 14 days after, ascites fluid of the mouse was collected and centrifuged, to thereby isolate a supernatant. The supernatant was admixed with the equiamount of buffer for adsorption (3-mol/L NaCl-1.5-mol/L Glycine—NaOH, pH: 8.5), followed by filtration. The filtrate was passed through Protein A Column (product of GE Healthcare Bioscience) which had been equilibrated with the buffer for adsorption, whereby an antibody was adsorbed by the column. Subsequently, the adsorbed antibody was eluted with 0.1-mol/L citrate buffer (pH: 3.0) from the column, whereby the anti-LR11 monoclonal antibody (A2-2-3 antibody) was purified.

### Example 2: Production of RAP/GST fusion protein

*Escherichia coli* DH5α, which had been transformed with a vector pGEX2T incorporating a human RAP gene (product of GE Healthcare Bioscience), was cultivated, and cells were recovered through centrifugation. The cells thus recovered from 3L culture liquid were suspended in a phosphate buffer (pH 7.2) containing lysozyme and a surfactant (Triton X-100), and broken through ultrasonication. The RAP/GST fusion protein contained in the centrifugation supernatant of the broken cell liquid was passed through Glutathione Sepharose 4 FF (product of GE Healthcare Bioscience) (10 mL), to thereby adsorb the protein onto the resin, followed by washing with phosphate buffer (PBS, pH 7.2), whereby a RAP-Sepharose resin was prepared.

### Example 3: Detection of soluble LR11 in sera collected from normal mammals

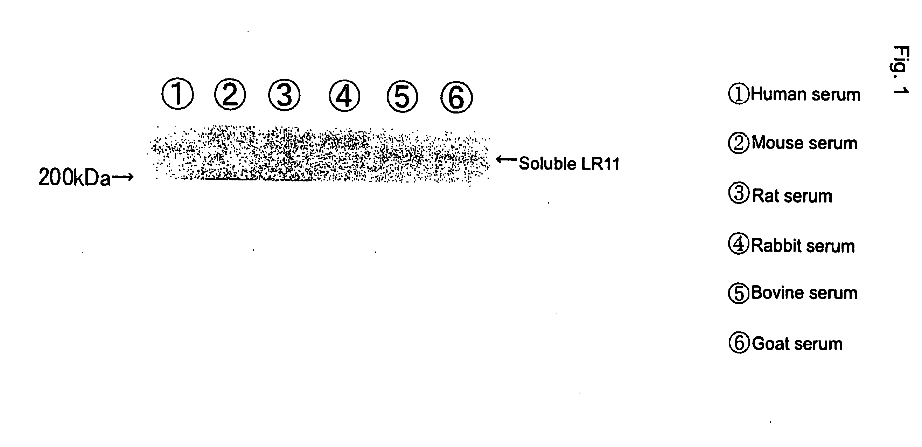

To each (3 mL) of the normal serum samples collected from various mammals, the RAP-Sepharose resin (30 µL) prepared in Example 2 was added, and the mixture was stirred at room temperature for two hours. Thereafter, the resin was washed with PBS. An equiamount of Tris buffer containing sodium dodecyl sulfate (SDS) was added to the thus-washed resin (30 µL), and the mixture was heated at 100°C for five minutes, followed by centrifugation. The obtained supernatant was subjected to 2 to 15% SDS-PAGE. The obtained protein was transferred to a PVDF membrane, and immunostained. The transfer membrane was blocked with PBS (BSA-PBST, pH 7.2) containing 1% bovine serum albumin (BSA) and 0.1% Tween (registered trademark) 20, and reacted with the anti-LR11 monoclonal antibody (A2-2-3) produced in Example 1, at room temperature for one hour. The membrane was thoroughly washed with 0.1% PBS (PBST, pH 7.2) containing Tween 20 and labeled with peroxidase by means of Vectastain ABC kit (Mouse) (product of Vector laboratories), followed by color development with hydrogen peroxide and diaminobenzidine. As a result, soluble LR11 having a molecular weight of 200 kDa or higher was confirmed to be present in sera of mammals such as human, mouse, rat, rabbit, bovine and goat (Fig. 1).

### Example 4: Comparative studies; Molecular weights of LR11 on human neuroblast cell (IMR32) membrane, free LR11, and soluble LR11 in human serum

### (1) Comparison of LR11 on cell membrane of IMR32 cells with free LR11 contained in culture supernatant of IMR32 cells

IMR32 cells were cultivated confluently in a 75-cm² culture dish to, and the culture supernatant and the cells were collected from the dish. The confluently cultured (75 cm²) cells were suspended in phosphate buffer (PBST, pH 7.2) (10 mL) containing a surfactant Triton (registered trademark) X-100, and broken through ultrasonication. The RAP-Sepharose resin (50 µL) prepared in Example 2 was added separately to the cell culture supernatant (40 mL) and to a centrifugation supernatant of broken cells (10 mL). Each mixture was stirred at room temperature for two hours, and the resin was washed with PBS. To the thus-washed resin (50 µL), equiamounts of β-mercaptoethanol and SDS-containing Tris buffer were added, and the mixture was heated at 100°C for 10 minutes, followed by centrifugation. The centrifugation supernatant was subjected to 2 to 15% SDS-PAGE. The obtained protein was transferred to a PVDF membrane, and immunostained. The transfer membrane was blocked with BSA-PBST, and reacted with the anti-LR11 monoclonal antibody (A2-2-3) produced in Example 1 at room temperature for one hour, and further reacted with a peroxidase-labeled anti-mouse IgG goat antibody at room temperature for one hour. The membrane was thoroughly washed with PBST, and reacted with a western blotting detection reagent ECL (product of GE Healthcare Bioscience). Detection was performed by use of an X-ray film.

### (2) Comparison of free LR11 contained in culture supernatant of IMR32 cells with soluble LR11 in human serum

IMR32 cells were cultivated in a 75-cm² culture dish confluently. The RAP-Sepharose resin (50 µL) prepared in Example 2 was added separately to the IMR32 cell culture supernatant (40 mL) and to a normal human serum (5 mL). Each mixture was stirred at room temperature for two hours, and the resin was washed with PBS. To the thus-washed resin (50 µL), equiamounts of β-mercaptoethanol and SDS-containing Tris buffer were added, and the mixture was heated at 100°C for 10 minutes, followed by centrifugation. The centrifugation supernatant was subjected to 2 to 15% SDS-PAGE. The obtained protein was transferred to a PVDF membrane, and immunostained. The transfer membrane was blocked with BSA-PBST, and reacted with the anti-LR11 monoclonal antibody (A2-2-3) produced in Example 1 at room temperature for one hour, and further reacted with a peroxidase-labeled anti-mouse IgG goat antibody at room temperature for one hour. The membrane was thoroughly washed with PBST, and reacted with a western blotting detection reagent ECL (product of GE Healthcare Bioscience). Detection was performed by sensitizing a film for instant photography.

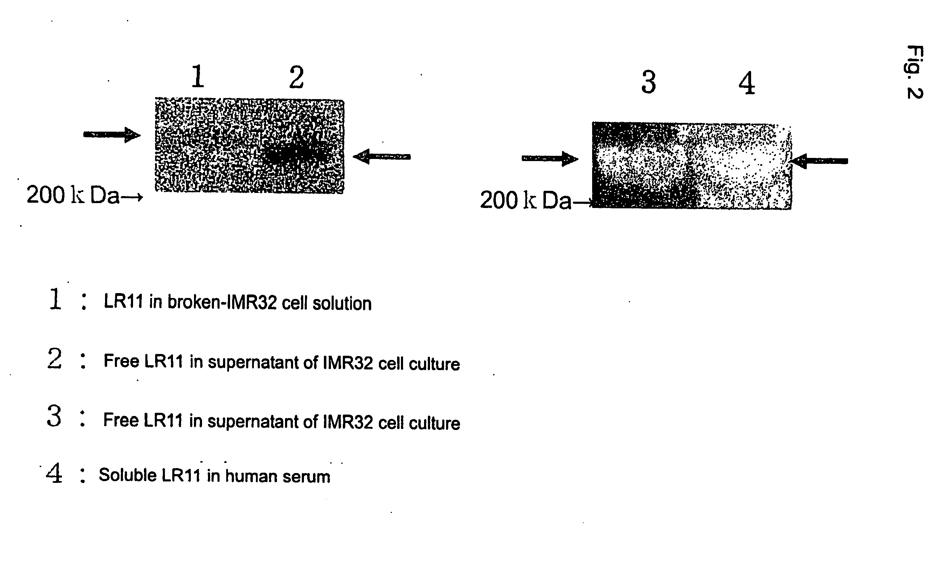

Fig. 2 shows the results of the above detection. As reported in Non-Patent Document 4, the free LR11 present in IMR32 cell culture supernatant (2 in Fig. 2) is shown to have a molecular weight slightly smaller than that of LR11 expressed on a surface of IMR32 cell membrane (1 in Fig. 2), and almost equivalent to that of soluble LR11 extracted from the human serum (4 in Fig. 2). Therefore, the free LR11 present in IMR32 cell culture supernatant and soluble LR11 in the human serum are thought to have a similar structure of full-length LR11 expressed on cells but lacking a C-terminal membrane-spanning domain.

### Example 5: Correlation between soluble LR11 and arteriosclerotic disease

The study was conducted on 96 people who were suspected of having an ischemic heart disease and underwent coronary angiography. According to the classifications by American College of Cardiology (ACC) and American Heart Association (AHA), the 96 people who had undergone coronary angiography were divided into the following groups: the coronary artery disease (CAD) group (62 people) including patients who had ≥50% stenosis (i.e., significant stenosis lesion) in any of the three coronary arteries (right coronary artery, left anterior descending coronary artery, and left circumflex coronary artery) observed in an coronary angiographic image, and patients having a past history of acute myocardial infarction; and the normal coronary artery group (NCA) (34 people) including patients who had no significant stenosis lesion in an coronary angiographic image. The tested people were also classified in terms of the number of lesion-observed vessels among three coronary arteries (i.e., the number of lesion vessel(s)) into the following groups: 0-vessel group (34 people, corresponding to NCA group), 1-vessel group (46 people), 2-vessel group (9 people), and 3-vessel group (7 people).
The serum soluble LR11 level was determined through the following procedure. Soluble LR11 was extracted in a manner similar to that of Example 3, employing serum (50 µL) and RAP-Sepharose gel (35 µL). After SDS-PAGE, the protein was transferred to a PVDF membrane, and detection was performed according to the method described in Example 4 (1). The detected bands were quantitated by NIH Image software. Using a concentration unit (U), which means an average concentration of detected bands attributed to soluble LR11 in plasma (20 µL) from each healthy subject, the soluble LR11 concentrations of subjects in the CAD group and in the NCA group were calculated.

In the test results, the average serum soluble LR11 levels of the CAD group and the NCA group were 5.0 ± 2.8 U and 3.8 ± 1.6 U, respectively, indicating that the CAD group had a soluble LR11 level significantly higher than that of the NCA group (p<0.05). Regarding the number of lesion vessel(s), the soluble LR11 level significantly increased as the number of lesion vessel increased from 0 to 2 (0-vessel group: 3.8 ± 1.6 U, 1-vessel group: 5.1 ± 2.6 U, 2-vessel group: 6.7 ± 4.2U, and 3-vessel group: 3.1 ± 1.7 U). In the 3-vessel group, the soluble LR11 level rather decreased. One possible reason for the decrease is that growth of vascular smooth muscle cells is suppressed under highly progressed arteriosclerosis, thereby lowering the soluble LR11 level.
Thus, the results suggest that detection of soluble LR11 allows to evaluate the presence or level of an arteriosclerotic disease and may provide an index for the progress of arteriosclerosis.

### Example 6: Correlation between soluble LR11 and intima-media thickness of carotid artery (IMT)

The study was conducted on 402 people suffering from abnormal lipidemea (LDL-cholesterol of >160 mg/dL, neutral fat of >200 mg/dL or HDL-cholesterol <35 mg/dL, and without diabetes, a thyroid disease or an endocrine disease).
The serum soluble LR11 level was determined through the following procedure. Soluble LR11 was extracted in a manner similar to that of Example 3, employing serum (50 µL) and RAP-Sepharose gel (35 µL). After SDS-PAGE, the protein was transferred to a PVDF membrane, and detection was performed according to the method described in Example 4 (1). The detected bands were quantitated by NIH Image software. Using a concentration unit (U), which means an average concentration of detected bands attributed to soluble LR11 in plasma (20 µL) of each healthy subject, the soluble LR11 concentrations of samples were calculated.
The IMT was measured by means of an ultrasonic scanner (SSD-1200CV, ALOKA).
As a result, the IMT had a significant correlation (p<0.001) to the serum soluble LR11 level with a correlation factor (R) of 0.48. Thus, the result suggests that detection of soluble LR11 allows to evaluate the presence or level of carotid IMT of abnormal lipidemea patients and may provide an index for the progress of arteriosclerosis.

## Claims

1. A method for evaluating the presence or level of an arteriosclerotic disease in a mammal or for evaluating a prophylactic or therapeutic effect on an arteriosclerotic disease in a mammal, comprising detecting soluble LR11 in a blood, serum or plasma sample collected from the mammal.

2. The method according to claim 1, wherein the mammal is a human.

3. The method according to claim 1 or 2, wherein the soluble LR11 is a protein having a structure of LR11 in which a C-terminal membrane-spanning domain is deficient.

4. The method according to any one of claims 1 to 3, wherein the soluble LR11 is detected by use of a substance having an affinity thereto.

5. The method according to claim 4, wherein the substance having an affinity to the soluble LR11 is an anti-soluble LR11 antibody and/or RAP.

6. Use of a kit for evaluating the presence or level of an arteriosclerotic disease or for evaluating a prophylactic or therapeutic effect on an arteriosclerotic disease, said kit comprising a substance which detects soluble LR11 and which has affinity to soluble LR11.

7. The use according to claim 6, wherein the substance which detects soluble LR11 is an anti-soluble LR11 antibody and/or RAP.

## Patentansprüche

1. Verfahren zur Evaluierung der Anwesenheit oder des Ausmaßes einer arteriosklerotischen Erkrankung in einem Säugetier oder zur Evaluierung einer prophylaktischen oder therapeutischen Wirkung auf eine arteriosklerotische Erkrankung in einem Säugetier, umfassend Detektieren von löslichem LR11 in einer Blut-, Serum-, oder Plasmaprobe, die von dem Säugetier genommen wurde.

2. Das Verfahren gemäß Anspruch 1, wobei das Säugetier ein Mensch ist.

3. Das Verfahren gemäß einem der Ansprüche 1 oder 2, wobei das lösliche LR11 ein Protein ist mit einer Struktur von LR11, in dem eine C-terminale membrandurchspannende Domäne fehlerhaft ist.

4. Das Verfahren gemäß einem der Ansprüche 1 bis 3, wobei das lösliche LR11 detektiert wird unter Verwendung einer Substanz, die Affinität dazu aufweist.

5. Das Verfahren gemäß Anspruch 4, wobei die Substanz, die Affinität zu löslichem LR11 aufweist, ein Antikörper gegen lösliches LR11 und/oder RAP ist.

6. Verwendung eines Kits zur Evaluierung der Anwesenheit oder des Ausmaßes einer arteriosklerotischen Erkrankung oder zur Evaluierung einer prophylaktischen oder therapeutischen Wirkung auf eine arteriosklerotische Erkrankung, wobei der Kitt eine Substanz enthält, die lösliches LR11 detektiert, und die Affinität zu löslichem LR11 aufweist.

7. Die Verwendung gemäß Anspruch 6, wobei die Substanz, die lösliches LR11 detektiert, ein Antikörper gegen lösliches LR11 und/oder RAP ist.

## Revendications

1. Procédé pour évaluer la présence ou le niveau d'une maladie artérioscléreuse chez un mammifère ou pour évaluer un effet prophylactique ou thérapeutique sur une maladie artérioscléreuse chez un mammifère, comprenant la détection de LR11 soluble dans un échantillon de sang, de sérum ou de plasma collecté du mammifère.

2. Procédé selon la revendication 1, dans lequel le mammifère est un humain.

3. Procédé selon la revendication 1 ou 2, dans lequel la LR11 soluble est une protéine ayant une structure de LR11 dans laquelle un domaine chevauchant la membrane C-terminal est déficient.

4. Procédé selon l'une quelconque des revendications 1 à 3, dans lequel la LR11 soluble est détectée par utilisation d'une substance ayant une affinité pour celle-ci.

5. Procédé selon la revendication 4, dans lequel la substance ayant une affinité pour la LR11 soluble est une RAP et/ou un anticorps anti-LR11 soluble.

6. Utilisation d'un kit pour évaluer la présence ou le niveau d'une maladie artérioscléreuse ou pour évaluer un effet prophylactique ou thérapeutique sur une maladie artérioscléreuse, ledit kit comprenant une substance qui détecte la LR11 soluble et qui a une affinité pour la LR11 soluble.

7. Utilisation selon la revendication 6, dans laquelle la substance qui détecte la LR11 soluble est une RAP et/ou un anticorps anti-LR11 soluble.
